(19) Europäisches Patentamt
European Patent Office
Office européen des brevets

(11) Publication number: **0 403 172 A2**

(12) # EUROPEAN PATENT APPLICATION

(21) Application number: **90306259.4**

(22) Date of filing: **08.06.90**

(51) Int. Cl.5: **C12N 9/90, C12N 15/61, C12P 21/00, C07K 7/04, C07K 15/04, //(C12N15/61, C12R1:465)**

(30) Priority: **12.06.89 US 364960**

(43) Date of publication of application: **19.12.90 Bulletin 90/51**

(84) Designated Contracting States: **AT BE CH DE DK ES FR GB GR IT LI NL SE**

(71) Applicant: **OKLAHOMA STATE UNIVERSITY**
**101 Whitehurst**
**Stillwater, Oklahoma(US)**

(72) Inventor: **Yu, Chang-An**
**4604 Fairfield Drive**
**Stillwater, Oklahoma 74074(US)**
Inventor: **Usui, Shigeyuki**
**105-4 North University Place**
**Stillwater, Oklahoma 74075(US)**

(74) Representative: **Tapping, Kenneth George et al**
**Erl Wood Manor**
**Windlesham Surrey, GU20 6PH(GB)**

(54) **Purified enzyme and process therefor.**

(57) Isopenicillin N epimerase is provided in purified form following isolation from cell-free extracts of Streptomyces clavuligerus. The enzyme of molecular weight of 47,000 (SDS-PAGE) and 50,000 (gel filtration) has been purified to electrophoretic homogeneity, is monomeric, and contains 1 mole of pyridoxal-5′-phosphate per mole of protein.

EP 0 403 172 A2

Xerox Copy Centre

# PURIFIED ENZYME AND PROCESS THEREFOR

This invention relates to enzyme technology. In particular, it relates to the enzyme isopenicillin N epimerase and to a process for preparing the enzyme in purified form.

The biosynthetic pathway through which the cephalosporin natural product, cephalosporin C, is produced is well understood. The pathway proceeds via the action of cyclase on the tripeptide δ-(L-α-aminoadipoyl)-L-cysteinyl-D-valine to form isopenicillin N. The latter is converted to penicillin N by the catalytic action of the enzyme known as epimerase. Epimerase is an important enzyme in the pathway since penicillin N serves as the substrate for deacetoxycephalosporin C synthetase (expandase), whereas isopenicillin N does not. Isopenicillin N epimerase has been demonstrated in extracts of Cephalosporium, acremonium, Jayatilake, G. S., et al., Biochem, J., 194, 645 (1981) and Streptomyces clavuligerus, Jensen, S. E., et al., Can. J. Biochem., 29, 1526 (1983); however, the crude enzyme was found to be too labile for further study.

This invention provides the enzyme isopenicillin N epimerase in highly purified form. It further provides a method for producing the enzyme in purified form from cell-free extracts of Streptomyces clavuligerus.

The enzyme is a monomeric protein having a molecular weight of 47,000 as determined by SDS-polyacrylamide gel electrophoresis and 50,000 as estimated by gel filtration.

The amino acid composition of the enzyme is shown below in TABLE 1.

The amino acid composition was determined by conventional as well as high performance liquid chromatographies as described hereinafter. As shown in the Table, the predominant residues of the enzyme are alanine, leucine and arginine.

TABLE 1

| Amino Acid Composition of Epimerase | |
|---|---|
| Amino Acid | Number of Residues Per 47,000 Daltons |
| Asx (Asp + Asn) | 29 |
| Thr | 29 |
| Ser | 22 |
| Glx (Glu + Gln) | 41 |
| Pro | 39 |
| Gly | 35 |
| Ala | 58 |
| Val | 21 |
| Cys | 4 |
| Met | 5 |
| Ile | 14 |
| Leu | 56 |
| Tyr | 3 |
| Phe | 14 |
| Lys | 3 |
| His | 9 |
| Arg | 47 |
| Trp | 5 |
| | 434 |

The 23-residue amino-terminal amino acid sequence of epimerase was determined by automated Edman degradation with a gas-phase sequencer. The following sequence was obtained: Ala-Val-Ala-Asp-Trp-Glu-Glu-Ala-Arg-Gly-Arg-Met-Leu-Leu-Asp-Pro-Thr-Val-Val-Asn-Leu-Asn-Thr.

The enzyme exhibits two absorption maxima at 280 nm and 420 nm, as shown by curve 1 of Figure 1 of the drawings. The absorption ratio $A_{280}/A_{420}$ is 7.59. The absorption coefficients are

$A^{1\%}_{280} = 8.96$ and $A^{1\%}_{420} = 1.18$

in 10 mM potassium phosphate, pH 7.0.

The isopenicillin N epimerase requires pyridoxal-5′-phosphate (PLP) to exhibit its full activity. The purified enzyme provided herein contains one mole of PLP per mole of enzyme and PLP is not removed from the enzyme by dialysis, gel filtration, or ion-exchange chromatography. The following results indicate that PLP is a bound cofactor for epimerase.

The appearance of the absorption maximum at 420 nm suggests that the formyl group of PLP forms an aldimine linkage with an amino group of the protein as shown below

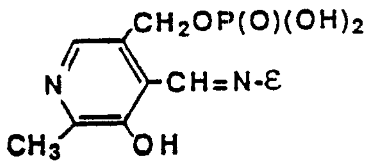

wherein ϵ represents the residue of epimerase protein. PLP bound to the epimerase was measured by the known phenylhydrazine method and an average value of one mole of PLP per 47,000 g of enzyme was obtained. The haloenzyme was converted to the apoenzyme by incubation at 0°C for 60 min with 10 mM potassium phosphate, pH 7.0, containing 10 mM hydroxylamine followed by gel filtration. The hydroxylamine treated epimerase expressed no activity in the absence of exogenous PLP and the absorption maximum at 420 nm disappeared (FIG. 1, curve 2). The epimerase activity was restored by the addition of PLP to the apoenzyme. The apparent Michelis constant for PLP was determined to be 2.4 μM. Reduction of epimerase (haloenzyme) with sodium borohydride destroyed catalytic activity and the addition of PLP did not restore the epimerase activity. Further, the reduced epimerase did not show the absorption maximum at 420 nm attributable to aldimine linkage of the epimerase (FIG. 1, curve 3). These results show that the aldimine linkage of the enzyme was reduced to the aldamine bond as shown below.

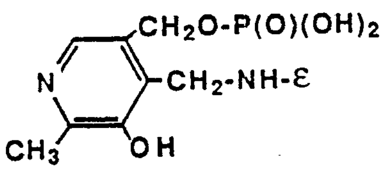

The following results obtained by the treatment of epimerase with fluorescamine further indicated that an amino group of epimerase forms the aldimine linkage with PLP, and that this linkage is involved in the active site of epimerase which is likely located inside the molecule.

Fluorescamine reacts with primary amino groups, such as the α-amino group of the N-terminal amino residue and the ϵ-amino groups of lysine in the enzyme to form fluorescent products with maximum excitation and emission wavelength of 390 nm and 475 nm, respectively. When the apoenzyme, prepared by treatment with hydroxylamine, was titrated with fluorescamine about 60% of the amino groups in epimerase reacted readily, indicating that these amino groups were likely to be located on the surface of the molecule. The epimerase activity still remained about 80% at the fluorescamine/epimerase ratio of 35 mole/mole, while with further addition of fluorescamine the activity was dramatically reduced. The fluorescamine treatment of epimerase is shown in FIG. 2 of the drawings which shows a plot of the emission fluorescence at 475 nm vs. activity.

The following 38-residue amino acid sequence was obtained from protease V8 digested epimerase and was bound to PLP: Gly-Ile-Thr-Thr-Val-Val-Asp-Gly-Ala-His-Ala-Pro-Gly-Phe-Leu-Asp-Leu-Asp-Leu-Ser-Arg-Ile-Pro-Cys-Asp-Phe-Tyr-Ala-Gly-Phe-Gly-His-Lys-Trp-Leu-Leu-Ala-Pro.

The following 11-residue amino acid sequence was also obtained from another tryptic peptide of epimerase: Leu-Pro-Pro-Gly-Thr-Asp-Ala-Ala-Glu-Leu-Arg.

Kinetic examination of the epimerase reaction indicates that the epimerase catalyzes the racemization of isopenicillin N and penicillin N. The epimerase has a $V_{max}$ of 3.93 μmole/min/mg and a $K_m$ of 0.30 mM for isopenicillin N, whereas it has a $V_{max}$ of 9.47 μmole/min/mg and a $K_m$ of 0.78 mM for penicillin N. When

these values are used, the calculated $K_{eq}$ for the racemization of isopenicillin N and of penicillin N is 1.08 which agrees well with the theoretical value of 1.0 for the following chemically symmetrical reaction.

Isopenicillin N (L-Form) ⇄ Penicillin N (D-form)

The kinetic behavior of the epimerase provided herein indicates that it is a racemase for isopenicillin N. The enzyme also could be referred to as an isomerase; however, we prefer to refer to the enzyme as epimerase.

The optimal pH for epimerase activity is about 7.8 to about 8.3 in 50 mM pyrophosphate-HCl buffer.

The activity of epimerase is partially stimulated by sulfhydryl reagents such as dithiothreitol (DTT) and 2-mercaptoethanol, while the chelating reagents and phenylmethylsulfonyl fluoride (PMSF) do not affect its activity. The effect of various sulfhydryl reagents on the activity of epimerase is shown below in TABLE 2.

TABLE 2

| Effect of Sulfhydryl Compounds on the Epimerase Activity | | |
|---|---|---|
| Compounds | Concentration (mM) | Activity (µmoles/min/ml) |
| No addition | -- | 150 |
| β-mercaptoethanol | 0.2<br>0.5 | 275<br>281 |
| Dithiothreitol | 0.2<br>0.5 | 290<br>300 |
| N-acetyl-L-cysteine | 0.2<br>0.5 | 224<br>190 |

The effect on the epimerase activity of various compounds recognized as inhibitors of other enzymes is shown below in TABLE 3.

TABLE 3

| Effect of Inhibitors on Epimerase | | |
|---|---|---|
| Compound | Concentration (mM) | Inhibition (%) |
| p-Chloromercuribenzoate | 0.1<br>1.0 | 70<br>88 |
| N-Ethylmaleimide | 0.1<br>1.0 | 26<br>45 |
| Iodoacetamide | 1.0 | 28 |
| Hydroxylamine | 0.1<br>1.0 | 80<br>95 |
| D-Cycloserine<br>Isoniazid<br>Iproniazid<br>FAD*<br>Phenylglyoxal | 1.0<br>1.0<br>1.0<br>0.5<br>1.0 | 23<br>23<br>25<br>80<br>33 |

*FAD = Flavin-Adenine Dinucleotide

The effect on epimerase activity of various substances which serve as a prosthetic group, cosubstrate,

or activator for other enzymes is shown below in TABLE 4.

TABLE 4

Effect of Various Compounds on Epimerase Activity

| Compounds[1] | Concentration (mM) | Activity (μmoles/min/ml) |
|---|---|---|
| No addition | -- | 229 |
| Ascorbate | 1 | 170 |
| $FeSO_4$ | 0.1 | 150 |
| ATP | 1 | 208 |
| FAD | 0.5 | 42 |
| α-Ketoglutarate | 1 | 208 |
| NADH | 1 | 184 |
| CoA | 1 | 202 |

[1]/ATP = Adenosine Triphosphate
NADH = Reduced Nicotinamide Adenine Dinucleotide
CoA = Coenzyme A

Because of the stimulation in epimerase activity by sulfhydryl reagents, the inhibitory effect of p-chloromercuribenzoate, N-ethylmaleimide and iodoacetamide (known sulfhydryl group modifiers) and the presence of cysteine (cystine) in epimerase, one or more of the sulfhydryl groups in the enzyme appear to be involved in the racemization reaction catalyzed by the epimerase.

The purified enzyme provided herein is substrate specific for isopenicillin N and penicillin N. The following TABLE 5 shows the results obtained with other substances evaluated as epimerase substrates.

TABLE 5

| Substrate Specificity of Epimerase | | |
|---|---|---|
| Compound | Concentration (mM) | Relative Activity (%) |
| Isopenicillin N | 1.4 | 100 |
| Penicillin N | 1.4 | 204 |
| IsoDAOC[1] | 1.4 | 0 |
| DAOC[2] | 1.4 | <1 |
| DAC[3] | 1.4 | 0 |
| Ceph C | 1.4 | 0 |
| ACV[4] | 1.4 | 0 |
| L-Amino adipic acid | 1.4 | 0 |
| D-Amino adipic acid | 1.4 | 0 |

[1]/IsoDAOC = Isodeacetoxycephalosporin C (2-cephem)
[2]/DAOC = Deacetoxycephalosporin C (3-cephem)
[3]/DAC = Deacetylcephalosporin C
[4]/ACV = δ-(L-α-aminoadipoyl)-L-cysteinyl-D-valine

The epimerase provided herein retains its activity in buffers having a range in pH of about 5.3 to about 10. For example, it is stable in such buffers as 50 mM sodium acetate, potassium phosphate, Tris-HCl, pyrophosphate-HCl, and carbonate-bicarbonate buffers.

The enzyme provided by this invention may be obtained from cell-free extracts of microorganisms which produce cephalosporin C, deacetoxycephalosporin C, deacetylcephalosporin C, cephamycin and like cephalosporin compounds. The enzyme may be obtained from the eukaryotic organisms, e.g., Cephalosporium acremonium (Acremonium crysogenum), and the prokaryotic organisms, e.g., Streptomyces clavuligerus, Streptomyces lipmanii, Streptomyces cattleya, and Nocardia lactamdurans (S. lactamdurans).

Many of such organisms are known and available, for example, Streptomyces clavuligerus ATCC 27064, NRRL 3585 and NRRL 3588. A particular microorganism from which the epimerase has been obtained is Streptomyces clavuligerus NRRL 18491. A culture of this microorganism has been deposited in the permanent culture collection of the Northern Regional Research Division, U.S. Department of Agriculture, Agricultural Research Service, Peoria, IL 61604, where it has been assigned the deposit number NRRL 18491.

The process for purifying epimerase described hereinafter is applicable to cell-free extracts containing the enzyme in varying concentrations. The amount of enzyme in the starting cell-free extract is not limiting if the enzyme is present in any reasonable amount.

This invention further provides a process for purifying isopenicillin N epimerase. According to the process, crude preparations of epimerase obtained from cell-free extracts of the enzyme via ammonium sulfate precipitation are chromatographed at pH 8.0 over a weakly anionic exchange resin such as derivatized cellulose resins, e.g., DEAE-cellulose (Whatman Inc., Clifton, NJ), DEAE-Sephacryl and DEAE-Sepharose (Pharmacia, Piscataway, NJ) or DEAE-Trisacyl (IBF Biotechnics, Villenueve-la-etarene, France). The enzyme is eluted from the resin with a linear salt gradient at pH 8.0. Following ultrafiltration and dialysis of combined active fractions, the active concentrate is chromatographed over affinity resin of the derivatized cellulose type such as diethylaminoethyl cellulose, e.g., DEAE Affi-Gel Blue (Bio-Rad, Richmond, CA). The enzyme is eluted from the resin with a linear salt gradient at pH 7, and the active fractions combined and concentrated by ultrafiltration. The concentrate is subjected to gel filtration at pH 6.0 by employing a gel of the polysaccharide type such as Sephadex G-200 (Pharmacia, Piscataway, NJ) in the presence of 0.01 mM pyridoxal-5′-phosphate. The enzyme-active fractions are combined, concentrated by ultrafiltration and the concentrate chromatographed over a calcium phosphate-cellulose resin or over hydroxylapatite at pH 6.0 again in the presence of 0.01 mM pyridoxal-5′-phosphate. The finely-divided calcium phosphate used can be prepared according to Jenner, J. L., U.S. Patent No. 3,737,516. The enzyme is eluted from the resin with a linear gradient of potassium phosphate at pH 6.0. The active fractions are combined, concentrated by ultrafiltration and dialyzed vs. pH 8.0 buffer containing 0.1 mM PLP.

The enzyme may be further purified via fast protein liquid chromatography on a strong anionic resin

such as Mono Q (Pharmacia, Piscataway, NJ) at pH 8.0 again in the presence of PLP.

The process is carried out at a temperature between about 0° C and about 5° C. A key feature of the process comprises the use of a series of buffers and chromatographic resins which allow separation of the epimerase from other proteins while maintaining the activity of the enzyme.

The crude enzyme preparation is obtained from wet whole cells of the producing organism by harvesting the cells from fermentation media in a conventional manner. The whole cells are homogenated in 10 mM pyrophosphate-HCl, pH 8.0, buffer containing 20% v:v of glycerol and 0.1 mM DTT. The suspension is sonicated at 0° C and centrifuged. The supernatant cell-free extract is then subjected to ammonium sulfate fractionation. The precipitate which is formed at an ammonium sulfate concentration of between about 35% and about 75% of saturation is collected and dialyzed against 10 mM pyrophosphate-HCl, pH 8.0, containing about 0.1 mM DTT and about 0.15 M sodium chloride, designated herein as Buffer A.

The dialysate of crude enzyme is chromatographed over the weak anionic exchange resin, preferably DE52, which equilibrated with Buffer A prior to use. The epimerase is eluted from the resin with a 0.15- 0.3 M sodium chloride linear gradient in Buffer A. The fractions off the column are assayed for epimerase activity in the assay method described hereinafter. Active fractions are combined, concentrated by ultrafiltration and dialyzed against 10 mM potassium phosphate, pH 7.0, containing about 0.1 mM DTT and 0.1 M sodium chloride (designated as Buffer B).

The dialyzed enzyme solution is next chromatographed over the affinity resin preferably DEAE Affi-Gel Blue (Bio-Rad Lab., Richmond, CA) which is equilibrated with Buffer B prior to use. Epimerase is eluted with the linear gradient 0.1-0.3 M NaCl in Buffer B. The active enzyme containing fractions are combined and concentrated by ultrafiltration. The concentrate is gel-filtered, e.g., through Sephadex-200 (Pharmacia Inc., Piscataway, NJ) which is equilibrated prior to use with 10 mM potassium phosphate, pH 6.0, containing 0.1 mM DTT, and 0.01 mM PLP (designated as Buffer C). The enzyme containing eluate fractions were combined, concentrated by ultrafiltration and the concentrate applied to a calcium phosphate-cellulose resin (Jenner, E. L., U.S. Patent No. 3,737,516) previously equilibrated with Buffer C. The column is washed with Buffer C and the epimerase is eluted with a linear gradient of 10-100 mM potassium phosphate, pH 6.0, in Buffer C. After combining active fractions and concentration via ultrafiltration, the enzyme concentrate is dialyzed against Buffer A containing 0.1 mM PLP.

At this stage of the process the epimerase is substantially purified and commonly possesses a specific activity of 2,700 nmol/min/mg with isopenicillin N as the substrate.

The enzyme is further purified by fast protein liquid chromatography (FPLC) on a strong anionic resin, preferably Mono Q. The resin is equilibrated prior to use with Buffer A containing about 0.01 mM PLP and is eluted from the resin with 0.15-0.45 M NaCl linear gradient in Buffer A containing PLP at a concentration of about 0.1 mM.

The level of purity of the epimerase obtained after the chromatography over the strong anionic resin is enhanced over the previous chromatographic step and is reflected in the increased specific activity. Commonly, the specific activity is about 3800 nmol/min/mg with isopenicillin N as the substrate.

In the above-described purification process, reducing agents other than DTT may be used in the buffer. For example, β-mercaptoethanol, N-acetyl-L-cysteine and like sulfhydryl reagents may be used. DTT is, however, the preferred additive.

The ultrafiltration of the chromatographic eluates is carried out in conventional manner solely for the purpose of reducing the volume of eluates for subsequent steps in the process. The purified epimerase provided by this invention is useful in converting isopenicillin N to penicillin N. The latter is the substrate for deacetoxycephalosporin C synthetase (expandase), e.g., the purified expandase provided by Yeh and Dotzlaf, U.S. Patent No. 4,753,881.

The 11, 23, and 38 amino acid residues of epimerase provided herein, in addition to being useful for cloning purposes, are useful as sources of amino acids for nutritional purposes, e.g., as feed supplements.

The purified epimerase provided herein has also been cloned as described by S. Kovacevic, et al., in copending application No. 89313150.8. The recombinant availability of the enzyme has the potential for providing the epimerase in greater abundance than from cell-free extracts.

The following Example and procedures are provided to further describe the invention and are not intended to be limiting thereof.

## Example 1

Isolation/Purification of Epimerase

A. Preparation of Cell-free Extract

Streptomyces clavuligerus, NRRL 18491, a cephamycin-C producing strain, was cultured in 500 ml Erlenmeyer flasks under the conditions described by Whitney, J. G., et al., Antimicrobial Agents and Chemotherapy, Vol. 1, pp. 247-251 (1972) and, after 48 h, cells harvested and frozen by conventional methods. The cells were harvested by centrifugation, washed first with 15 mM Tris-HCl, pH 7.5, buffer containing 1.0 M KCl and 20% (w/w) of ethanol and then with 15 mM Tris-HCl, pH 7.5. The washed cells were stored at -80°C until used.

Approximately 250 g of frozen whole cells of S. clavuligerus were thawed and homogenated in 10 mM pyrophosphate-HCl, pH 8.0 buffer containing 20% glycerol and 0.1 mM dithiothreitol to a total volume of one liter. The cell suspension was sonicated four times for 20 sec each time, and the sonicate was centrifuged at 30,000 g for 30 min. The supernatant was used as the cell-free extract in the following isolation procedure.

B. Isolation of Crude Epimerase

The cell-free extract was subjected to ammonium sulfate fractionation. The precipitate which formed between about 35% and about 75% of ammonium sulfate saturation was collected by centrifugation and redissolved in 10 mM pyrophosphate-HCl, pH 8.0 buffer containing 0.1 mM dithiothreitol and 0.15 M sodium chloride (Buffer A). The solution was dialyzed over about 15 h against the same buffer to provide crude epimerase.

C. Purification of Epimerase

The dialyzed crude solution of epimerase (250 ml) was applied to a DE52 column (3.7 x 25 cm) equilibrated with Buffer A. After washing the column with the same buffer, the epimerase was eluted with a linear gradient formed with 0.15 to 0.3 M sodium chloride in 10 mM pyrophosphate-HCl, pH 8.0, containing 0.1 mM dithiothreitol. Fractions showing enzyme activity were pooled, concentrated by ultrafiltration with an Amicon PM-10 membrane and then dialyzed for about 15 h against 10 mM potassium phosphate, pH 7.0, containing 0.1 mM dithiothreitol and 0.1 M sodium chloride (Buffer B).

The dialyzed solution (30 ml) was loaded onto a DEAE Affi-Gel Blue column (1.6 x 15 cm) which was equilibrated with Buffer B. The enzyme was retained on the column and was eluted with a linear gradient of 0.1 to 0.3 M sodium chloride in Buffer B. The enzyme containing fractions (assay) were combined and concentrated by ultrafiltration. The concentrate (200 ml) was loaded onto a Sephadex G-200 column (2.5 x 42 cm) equilibrated with 10 mM potassium phosphate, pH 6.0, containing 0.1 mM dithiothreitol and 0.01 mM pyridoxal-5′-phosphate (Buffer C). The enzyme containing fractions were combined, concentrated by ultrafiltration, and the concentrate (10 ml) loaded onto a calcium phosphate-cellulose column (1.6 x 10 cm) equilibrated with Buffer C. The column was washed with Buffer C and the epimerase eluted with a linear gradient of 10-100 mM potassium phosphate, pH 6.0 in Buffer C. The enzyme containing fractions were combined, concentrated by ultrafiltration and then dialyzed for about 15 h against Buffer A containing 0.1 mM PLP.

The solution of the enzyme (1 ml) was applied onto an FPLC Mono Q column (0.5 x 5 cm) equilibrated with Buffer A containing 0.01 mM PLP. The enzyme was eluted with 0.15-0.45 M sodium chloride linear gradient in Buffer A containing 0.01 mM PLP.

All steps in the above-described purification of epimerase were conducted at a temperature between about 0-4°C.

The progress of the purification was followed by assaying the material for epimerase activity obtained in each step of the isolation and purification. The following TABLE 6 shows the results obtained after each step.

TABLE 6

| Purification of Epimerase | | | | | | |
|---|---|---|---|---|---|---|
| | | Substrate | | | | |
| | | Isopenicillin N | | Penicillin N | | |
| Step | Protein[1] (mg) | Activity (nmol/min) | Specific Activity (nmol/min/mg) | Activity (nmol/min) | Specific Activity (nmol/min/mg) | Activity ratio of Racemization of Isopenicillin N and Penicillin N |
| Cell-free extract | 4430 | 23933 | 5.40 | 53516 | 12.1 | 2.24 |
| 35-70% $AmSO_4$ fraction | 2210 | 23680 | 10.7 | 50720 | 23.0 | 2.15 |
| DE52 eluate | 180 | 15074 | 83.7 | 27242 | 151 | 1.80 |
| DEAE-Affi gel blue eluate | 40.4 | 7972 | 197 | 16108 | 399 | 2.03 |
| Sephadex G-200 eluate | 9.75 | 6423 | 659 | 13077 | 1342 | 2.04 |
| Calcium phosphate eluate | 1.89 | 5072 | 2684 | 10328 | 5464 | 2.04 |
| Mono Q eluate | 1.15 | 4422 | 3846 | 8934 | 7770 | 2.02 |

[1]/The total protein was determined by the method of Lowry, O. H., et al., J. Biol. Chem., 193, 265 (1951).

## Assay Method for Epimerase

The epimerase activity was determined by the product formation using isopenicillin N and penicillin N as substrates. Formation of isopenicillin N and penicillin N was quantitated by HPLC. The standard assay mixture for the measurement of the racemization of isopenicillin N and penicillin N contained 1.4 mM isopenicillin N or 1.4 mM penicillin N, 0.2 mM dithiothreitol, 0.1 mM PLP, 50 mM pyrophosphate-HCl, pH 8.3 buffer, and enzyme in a final volume of 0.5 ml. The reaction mixture was incubated at 37° C for 20 min. The reaction was terminated by placing the reaction vessel in boiling water for 10 min, immediately cooling the reaction mixture with ice, and then filtering the cold mixture through a 0.45 µm filter.

Because of the difficulty in separating isopenicillin N and penicillin N in the assay samples via HPLC, both compounds were derivatized with o-phthaldialdehyde according to the method of Aswad, D. W., Anal. Biochem., 137, 405 (1984) and Usher, J., et al., Anal. Biochem., 149, 105 (1985). The derivatives were then analyzed by HPLC. The assay samples were prepared for HPLC as follows. The assay filtrate, obtained as described above, 20 µl was mixed with 5 µl of a derivatizing reagent consisting of 4 mg of o-phthaldialdehyde dissolved in 300 µl of methanol, 250 µl of 0.4 M sodium borate, pH 9.4, 390 µl of water and 60 µl of 1 M N-acetyl-L-cysteine, adjusted to pH 5-6 with sodium hydroxide. After a 2 min reaction time, 200 µl of 50 mM sodium acetate, pH 5.0, were added to the mixture and 50 µl aliquots were used for HPLC analysis.

The HPLC system used was a Varian HPLC system including a Vista 5560 unit with a Fluorichrom fluorescence detector and a model 4270 integrator (Varian Associates, Sugar Land, TX) and a model 230401 auto-sampling injector (Gilson Medical Electronics, Middleton, WI).

The derivatized isopenicillin N and penicillin N were separated and quantitated with the external standard by a Cyclobond I column (0.46 x 25 cm) (Astec Inc., Whippany, NJ) with mobile phase of 59% (v/v) 5 mM sodium phosphate-1% (v/v) 1M sodium acetate-40% (v/v) methanol, pH 6.0. A flow rate of 1 ml/min was used and total fluorescence (Ex 360 cm) was determined.

## Adsorption Spectrum

The absorption spectra shown in FIG. 1 of the drawings were obtained as follows. The epimerase (haloenzyme), curve 1, was treated with 10 mM hydroxylamine (apoenzyme), curve 2, or was reduced with 1 mM sodium borohydride, curve 3. After removal of hydroxylamine or sodium borohydride by gel filtration, absorption spectra were run in 10 mM potassium phosphate, pH 7.0, at the enzyme concentration of 0.57 mg/ml with an Aminco DW-2000 spectrophotometer.

## Amino Acid Sequence and Composition

Amino acids were analyzed by the methods of Heinrikson, R. L., and Meredith, S. C. (1984) Anal. Biochem. 136, 65-74, and Bidlingmeyer, B. A., et al., (1984) J. Chromatog. 336, 93-104, with HPLC reversed-phase after derivatization with phenylisothiocyanate (PITC) to phenylthiocarbamoyl amino acids (PTC-amino acids)..

The purified epimerase was extensively dialyzed against water and lyophilized in acid-washed tubes and then hydrolyzed with 6N HCl at 110° for 24 h. The hydrolysate was reacted with PITC and the PTC-amino acids formed were quantitated by HPLC with external amino acid standards using Ultrosphere-ODS column (0.46 cm x 25 cm) (Beckman, San Ramon, CA). The PTC-amino acids were eluted with the gradient of solvents (A) 50 mM ammonium acetate, pH 6.0, and (B) a mixture of acetonitrile-methanol-0.22 M ammonium acetate, pH 6.0, (44:10:46). Additionally, the amino acid composition of the epimerase was analyzed by conventional ninhydrin procedure.

The amino acid sequence analysis was carried out by automated Edman degradation using a model 470A gas phase protein sequencer with on-line detection of the released amino acid phenylthiohydantoin derivative (PTH-amino acids). The derivatives were detected by a model 120A PTH-amino acid analyzer of Applied Biosystems, Foster City, CA, Applied Biosystems Protein Sequencer User Bulletin No. 12 (1985). The purified epimerase, which was extensively dialyzed against water and lyophilized, was dissolved in 5% acetonitrile containing 0.1% trifluoroacetic acid and was then absorbed into the polybrene-coated glass

microfiber filter.

Fluorescamine Fluorescence

The emission fluorescence of fluorescamine treated epimerase shown in FIG. 2 of the drawings was obtained as follows. The epimerase (0.5 mg/ml) was incubated in 10 mM potassium phosphate, pH 7.0, containing 10 mM hydroxylamine at 0° C for 60 min. After removal of hydroxylamine by gel filtration, the treated enzyme (0.16 mg/ml) in 10 mM potassium phosphate, pH 7.0, was titrated with the indicated amounts of fluorescamine (50 mM) dissolved in dry acetone at 25° C. The sample was excited at 390 nm and the emission fluorescence was followed at 475 nm. The curve labeled by "O" shows the observed emission intensities. Simultaneously, aliquots of the enzyme solution were withdrawn and activity for isopenicillin N was assayed. The assay results (% activity) are shown on the curve labeled "●" in FIG. 2.

Molecular Weight Determination

The molecular weight of isopenicillin N epimerase by sodium dodecylbenzenesulfate polyacrylamide gel electrophoresis (SDS-PAGE) was carried out as follows. The purified epimerase, 10 µg, treated with 2% SDS and 5% β-mercaptoethanol, was applied to 12.5% gel. The electrophoresis was performed with the protein standards of Weber, K., and Osborn, M. (1969) J. Biol. Chem. 244, 4406-4412. The protein standards used were (1) phosphorylase B (92,500), (2) bovine serum albumin (66,200), (3) ovalbumin (45,000), (4) carbonic anhydrase (31,000), (5) soybean trypsin inhibitor (21,500), and (6) lysozyme (14,400).

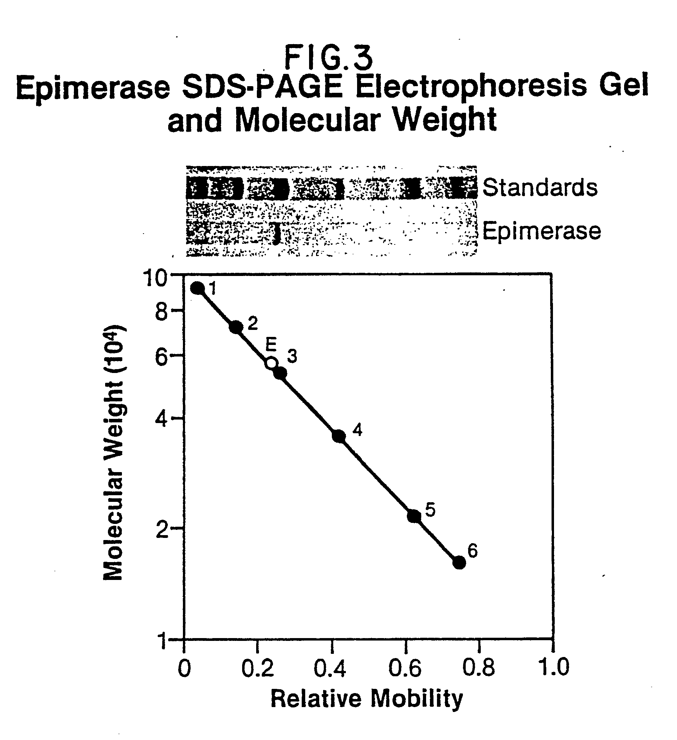

FIG. 3 of the drawings is a semilogarithmic plot of molecular weight against mobility on the gel plate. The numbers in the plot indicate the protein standards used as shown below in parentheses. E designates epimerase.

The following is a description of the method used for cloning epimerase in Escherichia coli and its expression therefrom.

A. Culture of E. coli K12 RR1ΔM15/pOW390

A lyophil of E. coli K12 RR1ΔM15/pOW390 can be obtained from the Northern Regional Research Laboratories (NRRL), Peoria, IL 61604, under the accession number NRRL B-18431 and used directly as the "culture" in the process described below.

One liter of TY broth (8 g tryptone, 5 g NaCl, and 5 g yeast extract per liter) containing 100 µg/mL ampicillin was inoculated with a culture of E. coli K12 RR1ΔM15/pOW390 and incubated with aeration at 37° C overnight (15-18 hours). The resulting culture was used as a source of plasmid pOW390.

B. Isolation of Plasmid pOW390

The culture prepared in Example 1A was centrifuged at 5200 rpm for 10 minutes at 4° C to pellet the cells. The resulting supernatant was discarded. The cell pellet was resuspended in 28 mL of a solution of 25% sucrose and 50 mM EDTA. About 1 mL of a solution of 20 mg/mL lysozyme in 50% glycerol and 0.25 M Tris-HCl, pH = 8.0, and about 1.5 mL of 0.5 M EDTA, pH = 8.0, were added to and mixed with the cell suspension. The resulting mixture was incubated on ice for 15 minutes. Three mL of lysing solution (prepared by mixing 3 mL of 10% Triton X-100; 75 mL of 0.25 M EDTA; pH = 8.0; and 7 mL of water) were added to the lysozyme-treated cells with gentle mixing. The resulting solution was incubated on ice for another 15 minutes.

The cellular debris was removed from the solution by centrifugation at 17,000 rpm for about 45 minutes at 4° C. About 28.6 g of CsCl and ~1 mL of a 5 mg/mL ethidium bromide solution were added to the ~30 mL of supernatant. Then, the volume was adjusted to 40 mL with water and the solution decanted into an ultracentrifuge tube. The tube was sealed, and the solution was centrifuged at 49,500 rpm for ~18 hours. The resulting plasmid band, visualized with ultraviolet light, was isolated, extracted with CsCl-saturated isopropanol to remove the ethidium bromide, and dialysed against three changes of ~20 volumes of TE buffer (10 mM Tris-HCl, pH = 7.5, and 1 mM EDTA). The dialysate was collected; then, two volumes of ethanol and 0.05 volumes of 3 M sodium acetate solution were added. The ethanol mixture was cooled to

-20° C, and the plasmid DNA was pelleted by centrifugation at 10,000 rpm for 30 minutes at -10° C. The resulting pellet was resuspended in ~1 mL of TE buffer and then extracted with an equal volume of a phenol:chloroform mixture (1:1, v/v). The DNA in the aqueous phase was recovered by the addition of 0.1 volume of 3 M NaOAc and 2 volumes of ethanol, followed by incubation at -20° C for ~30 minutes and centrifugation at 15,000 rpm for 20 minutes. The resulting DNA pellet was rinsed first with 70% ethanol and then with 100% ethanol and dried.

The ~1.5 mg of plasmid pOW390 DNA obtained by this procedure was suspended in 1.5 mL of 0.1X TE buffer and stored at -20° C. A restriction site and function map of plasmid pOW390 is presented in Figure 1 of the accompanying drawings.

## Example 2

## Construction of Plasmid pOW392

### A. Construction of Plasmid mOW390

Identical plasmid constructs can be achieved employing different methods and sources of gene sequences. Plasmid mOW390 was constructed by ligating the ~0.9 kb StyI-BamHI restriction fragment of plasmid pOW390, prepared as noted in B., below, with HincII-BamHI-digested, replicative form (RF) M13 vector, prepared as noted in C., below. The ~0.9 kb StyI-BamHI fragment contains a large portion of the racemase gene, including the 5′-end, but not the 3′ end. The StyI end was "filled in" so as to make it blunt-ended, rendering the end amenable to ligation with the blunt-ended HincII end. The plasmid pOW390 clone originated from cosmid pOW379, derived from a Streptomyces clavuligerus genomic cosmid library. Cosmid pOW379 was identified by hybridization using a "guessmer" DNA probe designed on the basis of the amino-terminal amino acid residue sequence of purified S. clavuligerus racemase and species codon-usage bias. The desired phage M13 clone can be identified using the "guessmer" probe in a plaque hybridization procedure or by restriction enzyme analysis. Because of the present invention, however, the construction of mOW390 is greatly simplified, primarily because plasmid pOW390 can be used as the source of the S. clavuligerus racemase gene. The M13-derived plasmid mOW390 was a useful intermediate in the site-specific mutagenesis carried out to create an NcoI restriction enzyme recognition site at the 5′ end of the S. clavuligerus racemase coding sequence.

### B. Isolation of the ~0.9 kb BamHI-StyI Restriction Fragment from Plasmid pOW390

Approximately 25 µg of the plasmid pOW390 DNA in 25 µl 0.1X TE buffer, as prepared in Example 1B, were added to and mixed with 40 µl of 10X StyI buffer (1.0 M NaCl; 500 mM Tris-HCl, pH = 8.0; and 100 mM $MgCl_2$), 335 µl of glass-distilled water, and 5 µl (~50 units) of restriction enzyme StyI. Unless otherwise noted, restriction enzymes were obtained from New England Biolabs, 32 Tozer Road, Beverly, MA 01915. Unit definitions herein correspond to the particular manufacturer's unit definitions. The resulting reaction was incubated at 37° C for 90 minutes. The StyI ends were then made blunt ("filled-in") by the method of Maniatis et al., Molecular Cloning: A Laboratory Manual, Cold Spring Harbor Laboratory, 1982, pp. 113-114. The reaction was then extracted with phenol and chloroform, and the StyI-digested plasmid pOW390 DNA was precipitated with NaOAc and ethanol and then resuspended in 45 µl of 1X BamHI buffer (100 mM NaCl; 10 mM Tris-HCl, pH = 7.5; 10 mM $MgCl_2$). Five µl (~50 units) of restriction enzyme BamHI were added and the mixture incubated at 37° C for 90 minutes. The reaction was then extracted with phenol and chloroform, and the BamHI-StyI (blunt)-digested plasmid pOW390 was precipitated with NaOAc and ethanol and then resuspended in 9 µl of $H_2O$. About 1 µl of loading buffer (25% v/v glycerol, 0.05% w/v bromphenol blue, and 0.05% xylene cyanol) was added to the solution of DNA, which was then electrophoresed on a 1% agarose gel until the desired ~0.9 kb BamHI-StyI restriction fragment was clearly separated from the other digestion products.

The electrophoresed DNA was visualized by staining the gel in a dilute solution (0.5 µg/ml) of ethidium bromide and exposing the stained gel to long-wave UV light. After the fragments were located, a small slit

was made in the gel in front of the ~0.9 kb fragment, and a piece of Schleicher and Schuell (Keene, NH 03431) DEAE membrane was placed in the slit. Upon further electrophoresis, the DNA non-covalently bound to the DEAE membrane. After the desired fragment was bound to the DEAE membrane, the membrane was removed and rinsed with low salt buffer (100 mM NaCl; 0.1 mM EDTA; and 20 mM Tris-HCl, pH = 8). Next, the membrane was placed in a small tube and immersed in high salt buffer (1 M NaCl; 0.1 mM EDTA; and 20 mM Tris-HCl, pH = 8) and then incubated at 65° C for 10 minutes to remove the DNA from the DEAE paper. After the 65° C incubation, the incubation buffer was collected, and the membrane was rinsed with high salt buffer. The rinse solution was pooled with the incubation buffer before collecting the desired DNA fragments.

The volume of the high salt-DNA solution was adjusted so that the NaCl concentration was 0.25 M, and then three volumes of cold, absolute ethanol were added to the solution. The resulting solution was mixed and placed on ice for 10-20 minutes. The solution was then centrifuged at 15,000 rpm for 15 minutes. After another precipitation to remove residual salt, the DNA pellet was rinsed with 70% ethanol, dried, resuspended in 20 µl of TE buffer, and constituted the desired restriction fragment. About 0.2 µg of the ~0.9 kb fragment was obtained.

C. Preparation of BamHI-HincII-Digested Vector M13mp18 RF DNA and Construction of Plasmid mOW390

About 2.5 µg of M13mp18 RF DNA (available from New England Biolabs (NEB)) were digested in 100 µl of BamHI buffer with 1 µl (~20 units) of restriction enzyme BamHI for 90 minutes at 37° C. The reaction mixture was extracted with phenol:chloroform and the DNA, in the aqueous phase, concentrated by ethanol precipitation.

The BamHI-digested M13mp18 was resuspended in 100 µl HincII buffer (10 mM Tris-HCl, pH = 7.4, 7 mM $MgCl_2$, and 1 mM DTT) with 1 µl (~10 units) of restriction enzyme HincII for 90 minutes at 37° C. The reaction was extracted and precipitated as above and resuspended in 20 µl 0.1 X TE buffer and constituted ~2 µg of the desired BamHI-HincII digested vector. The other BamHI-HincII fragment was only ~10 bp and was not precipitated. It did not interfere with the ligation.

Two µl of the ~0.9 kb BamHI-StyI restriction fragment of plasmid pOW390 from B., above, and 1 µl of BamHI-HincII-digested vector M13mp18 were ligated in a 20 µl reaction containing the DNA fragments, 2 µl of 10X ligase buffer (0.5 M Tris-HCl, pH 7.5, and 100 mM $MgCl_2$), 2 µl of 5 mM ATP, 1 µl of 6 µg/µl BSA, 12 µl of glass-distilled water, and 1 µl (1 Weiss unit) of T4 DNA ligase (NEB). The "filled-in" StyI ends were ligatable with the HincII blunt ends. The reaction was incubated ~18 hours at 15° C. The ligated DNA constituted the desired plasmid mOW390 along with other ligation products.

Competent E. coli K12 JM109 ("Epicurean Coli™") were purchased from Stratagene (3770 Tansy Street, San Diego, CA 92121) and transformed with a ligation reaction mixture constituting plasmid mOW390 in substantial accordance with the manufacturer's directions, except that the DNA was in a volume of 20 µl and no dilution into medium or expression time was necessary. Post-transformation, the cells were distributed in ~1, 10, 20, 40 and 50 µl samples to 13 X 100 mm sterile glass tubes containing 0.25 mL/tube E. coli K12 JM109 in logarithmic growth phase. To these tubes were added 3 mL of top agar (L broth with 0.8% agar kept molten at 45° C). The cell-top agar mixture was then plated on L-agar plates containing 40 µg/mL 5-bromo-4-chloro-3-indolyl-β-D-galactoside (X-gal) and 0.1 M isopropylthio-β-galactoside (IPTG), and the plates were incubated at 37° C overnight. (For more detailed descriptions and explanations of M13 procedures, see M13 Cloning/Dideoxy Sequencing Instruction Manual, Bethesda Research Laboratories (BRL), Life Technologies, Inc., Gaithersburg, MD 20877.) Transformants are identified by insertional inactivation of β-galactosidase activity (colorless plaque phenotype) and restriction enzyme analysis of replicative form (RF) DNA. For screening purposes, clear plaques are plugged from the plate overlay with a Pasteur pipette into 3 mL per plaque of early logarithmic growth phase E. coli K12 JM109. Cultures are incubated from 6 to 18 hours at 37° C with aeration.

Following this incubation, 1.5 mL of each culture are pelleted in separate 1.5 mL Eppendorf tubes. The supernatants are decanted into fresh tubes and stored at 4° C to serve as a source of phage inoculum. Replicative form DNA is prepared from the cell pellets in substantial accordance with the teaching of the alkaline plasmid preparation procedure of Birnboim and Doly, 1979, Nuc. Acid Res. 7(6): 1513-1523, with the following exceptions. The procedure is scaled up such that 1.5X volumes of Solutions I, II, and III are used, and the cleared lysate is extracted once with an equal volume of $CHCl_3$. The DNA is then precipitated by the addition of 0.4 volumes of isopropanol and incubation at room temperature for 20 minutes. The DNA is collected by centrifugation and then precipitated with ethanol out of 0.3 M NaOAc. The DNA isolated from individual plaques was analyzed for the presence of an insert by EcoRI-HindIII restriction enzyme digestion.

The orientation of the fragment was pre-determined by the compatible ends (BamHI-BamHI, "filled-in" StyI-HincII). By this method, E. coli K12 JM109/mOW390 cells were identified; these cells were then used as a source of plasmid mOW390 for the site-specific mutagenesis, as described below.

D. Preparation of Single-Stranded Plasmid mOW390 DNA and Site-Specific Mutagenesis to Construct Plasmid mOW391

A 10 mL culture of early logarithmic growth phase E. coli K12 JM109 was inoculated with ~200 μl of phage stock (prepared in Example 2C) and incubated ~18 hours at 37° C with aeration. The culture was cen trifuged and the resulting supernatant transferred to a new tube and recentrifuged. The supernatant was again decanted to a fresh tube. One mL of a solution of 25% polyethylene glycol (molecular weight ≅ 3,350) in 3 M NaCl was added to the supernatant, which was then incubated for 15 minutes at room temperature. The resulting mixture was centrifuged for 30 minutes at 10,000 rpm. The pellet obtained by the centrifugation contained the single-stranded plasmid mOW390 and was resuspended in 400 μl of TE buffer. The solution was extracted first with $CHCl_3$ and then with TE-saturated phenol. The phenol was allowed to stay in contact with the aqueous phase for 15 minutes. The solution was then extracted twice with a mixture of TE-saturated phenol:$CHCl_3$ (1:1, v/v), and twice with $CHCl_3$ alone. The DNA was then precipitated out of 0.3 M NaOAc, collected by centrifugation, and the resulting pellet resuspended in 100 μl of 0.1X TE buffer. This solution constituted ~5 μg of single-stranded plasmid mOW390 DNA.

E. Mutagenesis

The single-stranded DNA fragments used in the mutagenesis (and subsequent hybridizations to detect desired phages) were synthesized on an automated DNA synthesizer, with the exception of the M13 universal primer (a 15-mer), which was purchased from BRL. The mutagenesis fragments were designated as follows: (1) RACEATG, a single-stranded DNA 40 nucleotides in length that is homologous to the racemase coding sequence in plasmid mOW390 except for 1 base, the mismatch (underlined) of which will create a restriction enzyme NcoI recognition sequence at about position 1 of the racemase coding sequence, with the DNA sequence:

NcoI
5'-GCGGGAGATGCGTTTGCCATGGCGGTAGCCGACTGGGAAG-3'

(2) RACE-17, a single-stranded DNA 17 nucleotides in length that is merely a subfragment of RACEATG with the DNA sequence:

NcoI
5'-TGCGTTTGCCATGGCGG-3'

The 5′ ends of about 100 pmols of RACEATG were phosphorylated (kinased) in a reaction mixture containing single-stranded DNA at a concentration of 1 pmol/μl, 10 μl of 10X ligase buffer, 1000 pmols adenosine triphosphate (ATP), 10 μl of 0.1 M DTT, 65 μl of glass-distilled water, and 1 μl (10 Richardson units) of T4 polynucleotide kinase (Boehringer-Mannheim Biochemicals, (BMB) 7941 Castleway Drive, P.O. Box 50816, Indianapolis, Indiana 46250). The reaction mixture was incubated at 37° C for 30 minutes, at which time an additional 1 μl of enzyme was added. The reaction mixture was then incubated for another 30 minutes at 37° C and then quenched by incubation at 68° C for 5 minutes. The 5′ ends of about 40 pmols of M13 universal primer were kinased in an analogous 40 μl of reaction mixture containing the same amount of enzyme.

The single-stranded plasmid mOW390 DNA was mutagenized in substantial accordance with the teaching of Adelman et al., 1983, DNA 2(3): 183-193 as described below. The annealing reaction was carried out by adding ~500 nanograms (in 15 μl of 0.1X TE buffer) of single-stranded plasmid mOW390 DNA to 8 μl of 10X annealing buffer (100 mM Tris-HCl, pH = 7.5; 1 mM EDTA; and 500 mM NaCl), 4 μl (4 pmols) of kinased RACEATG, 4 μl (4 pmols) of kinased M13 universal sequencing primer, and 50 μl of water, incubating the mixture at 80° C for 2 minutes, then at 55° C for 5 minutes, and finally at room

temperature for 5 minutes.

The extension reaction was carried out by adding 120 µl of the following mixture to the solution of annealed DNA: 20 µl 10X Klenow-ligase buffer (100 mM Tris-HCl, pH = 7.5; 1 mM EDTA; and 500 mM NaCl), 20 µl of 0.1 M DTT; 20 µl of a solution 6.25 mM in each of dGTP, dATP, TTP, and dCTP; 20 µl of 5 mM ATP; 120 µl of water; and 2.5 µl (12.5 units) of Klenow enzyme (BMB). The extension reaction mixture was incubated at room temperature for 1 hour, then at 37° C for 4 hours, and finally at 14° C for ~18 hours.

The extension reaction mixture was extracted once with $CHCl_3$ and the DNA precipitated with ethanol and NaOAc and collected by centrifugation. The DNA pellet was resuspended in 400 µl 1X S1 buffer (0.3 M NaCl and 3 mM $Zn(OAc)_2$). Half the DNA solution was held in reserve at -20° C; half was aliquoted to five 1.5 mL tubes. To four of these tubes was added 1 µl of S1 nuclease (BMB) that had been diluted to 200 30-minute units per µl. The reactions were incubated at room temperature for 5, 10, 15, and 20 minutes, respectively. The reactions were stopped by first adding 5-10 µg of tRNA to the reaction mixture to serve as carrier, then extracting with a TE-saturated phenol-$CHCl_3$ mixture (1:1, v/v). The sample that was not treated with S1 (the negative control) was also extracted. The DNA in the aqueous phase was concentrated by ethanol precipitation and collected by centrifugation. The DNA pellets were each resuspended in 20 µl water.

Ten µl of each of the resulting S1-treated DNA solutions were used to transform E. coli K12 JM109 in substantial accordance with the procedure described in Example 2B, except that the plates did not contain either X-Gal or IPTG. Desired mutants were identified by hybridization of radiolabelled oligonucleotide RACE-17 with plasmid DNA blotted onto nitrocellulose filters as described below.

After plaque formation, the plates were incubated at 4° C for ~1 hour to harden the top agar. Nitrocellulose filters were placed on top of the lawn of each of two plates, containing ~50-200 plaques, from each of the negative control, the 10 minute S1-treated series, and the 20 minute S1-treated series. Contact between the filter and the surface of the lawn was maintained for ~1 minute, at which time the nitrocellulose filter was treated, by using saturated 3MMChr filter papers (Whatman LabSales, Inc., P.O. Box 1359, Hillsboro, Oregon 97123-1359), with 0.1 N NaOH-1.5 M NaCl for ~5 minutes, then 0.5 M Tris-HCl(pH = 7.0)-3 M NaCl for ~5 minutes. The nitrocellulose filters were air-dried and then baked in vacuo at 80° C for 30 minutes.

The nitrocellulose filters were prehybridized for ~5 minutes at room temperature in a solution of 6X SSC (20X SSC is 3 M NaCl and 0.3 M Na citrate), 10X Denhardt's solution (0.2 g of polyvinylpyrrolidone), 0.2 g of bovine serum albumin, and 0.2 g of Ficoll per 100 mL of water), 0.1% NaPPi, 0.1% SDS, and 10 µg/mL of denatured E. coli chromosomal DNA. The filters were then hybridized in a solution of 6X SSC, 10X Denhardt's solution, 0.1% NaPPi, and 1 pmol/5 mL of $^{32}$P-RACE-17. The $^{32}$P-RACE-17 was prepared by phosphorylating the 5′ ends of 100 pmols of RACE-17 in substantial accordance with the procedure described earlier in this example, except that ~70 pmol of $\gamma$-$^{32}$P-ATP (New England Nuclear (NEN), 549 Albany Street, Boston, MA, 02118, Catalog # NEG-002A) were used instead of non-radioactive ATP. After hybridization, the filters were rinsed twice for 5 minutes per wash in excess 6X SSC at room temperature, then at 52° C in excess 6X SSC for 20 minutes per wash. The filters were air-dried and autoradiographed for 2 hours at -70° C with a Quanta III® intensifying screen (Dupont, Instrument Products, Biomedical Division, Newtown, CN 06470). Desired mutants, those containing sequences complementary to the sequence of RACE-17, exposed the film due to binding of the radiolabelled oligomer by the plasmid DNA bound to the filter. The identity of a correct mutant, designated plasmid mOW391, was confirmed by restriction analysis of its RF DNA, which was prepared in substantial accordance with the procedure described in Example 2C.

F. Final Construction of Plasmid pOW392

The RF DNA of plasmid mOW391 contains the racemase coding sequence on the NcoI-BamHI restriction fragment utilized in the construction of the E. coli expression plasmid pOW392.

Replicative form DNA from plasmid mOW391-infected E. coli K12 JM109 was isolated in substantial accordance with the procedure described in Example 2C. About 10 pg of the RF DNA of plasmid mOW391 DNA were digested with restriction enzyme BamHI (~10 units) and NcoI (~10 units) in a reaction containing the DNA in 1X React® 3 buffer (500 mM Tris-HCl, pH = 8.0, 100 mM $MgCl_2$, 1 M NaCl; Bethesda Research Laboratories, P.O. Box 6009, Gaithersburg, MO 20877). After incubation for ~90 minutes at 37° C, the reaction mixture was subjected to agarose gel electrophoresis, and the ~0.9 kb BamHI-NcoI fragment was isolated in substantial accordance with Example 2B.

Plasmid pCZR111 is available in E. coli K12 JM109 from the NRRL under accession number B-18249.

It can be isolated in substantial accordance with Example 1 except that 15 µg/ml tetracycline is included in the culture medium rather than 100 µg/ml ampicillin. A restriction site and function map of pCZR111 is presented in Figure 2.

The vector was generated by digesting plasmid pCZR11 with XbaI and BamHI. About 1 µl of XbaI (~10 units) is added to 10 µl plasmid pCZR111 (~10 µg) and 5 µl 10X XbaI buffer (500 mM Tris-HCl, pH = 8.0, 100 mM $MgCl_2$, and 500 mM NaCl). After incubation at 37° C for 90 minutes, 0.5 µl of 5 M NaCl and 1 µl BamHI (~10 units) is added and the incubation continued at 37° C for an additional 90 minutes. The reaction mixture is then subjected to agarose gel electrophoresis, and the ~5.75 kb XbaI-BamHI vector fragment is isolated in substantial accordance with Example 2B.

An intermediate plasmid can be created by ligating the ~0.9 kb BamHI-NcoI fragment from mOW391, the ~5.75 kb XbaI-BamHI vector fragment from pCZR11 and a double-stranded XbaI-NcoI restriction fragment synthesized by the phosphotriester method to yield the ~6.7 kb intermediate plasmid. The double stranded DNA fragment has the following sequence:

```
5'-CTAGAGGGTATTAATAATGTATATTGATTTTAATAAGGAGGAATAATCC-3'
        |||||||||||||||||||||||||||||||||||||||||||||
        3'-TCCCATAATTATTACATATAACTAAAATTATTCCTCCTTATTAGGGTAC-5'
```

The ligation mixture is transformed into E. coli K12 JM109 in substantial accordance with Example 2C, and plasmid DNA isolated from transformants is analyzed by restriction enzyme digestion to confirm the presence of the correct inserts.

To complete the expression vector, an ~2 kb BamHI fragment was isolated from plasmid pOW390 in substantial accordance with Example 2B. This ~2 kb BamHI fragment comprises the 3′ coding region of the racemase gene. The completed vector, therefore, contains the entire coding region of the racemase gene.

The ~2 kb BamHI fragment (2.5 µl, ~0.5 µg) was ligated to the intermediate vector plasmid digested with BamHI in substantial accordance with the procedure described in Example 2C to form desired plasmid pOW392. A restriction site and function map is provided in Figure 3 of the drawings.

The ligation reaction constituting the desired plasmid pOW392 was transformed into competent E. coli K12 RR1ΔM15 (NRRL B-15440). Aliquots of the transformation mixture were plated on L-agar plates containing tetracycline (15 µg/mL). The plates were incubated at 37° C for ~18 hours. Tetracycline-resistant transformants were further screened by restriction enzyme analysis of their plasmid DNA to identify the desired plasmid pOW392 transformants. Plasmid DNA was prepared from 3 mL cultures in substantial accordance with the Birnboim and Doly procedure described above for preparing RF DNA from phage M13-infected E. coli K12 JM109 cell pellets. Plasmid pOW392 DNA from one transformant was prepared in substantial accordance with the procedure described in Example 1 for use in subsequent constructions. The plasmid was then transformed into E. coli K12 JM109 purchased from Strategene.

## Example 3

## Assay of E. coli-produced Racemase Activity

### A. Culture of E. coli K12 JM109/pOW392 for Expression of Racemase Activity

An E. coli K12 JM109/pOW392 transformant was grown at 30° C overnight in 500 ml of L broth (containing 15 µg/ml of tetracycline) in a gyrotory incubator. The cells were diluted 1:10 by adding 100 ml of the overnight culture to 900 ml of fresh medium containing 15 µg/ml tetracycline in a 2.8 L Fernbach flask and incubated a further hour at 30° C under the same growth conditions. The temperature of the air shaker was then raised to 42° C and incubation continued for an additional 6.5 hours. The cI857 temperature-sensitive repressor of the lambda pL promoter, positioned to drive racemase expression on plasmid pOW392, is inactivated at 42° C and so allows for expression of racemase. After induction, the cells were harvested by centrifugation and used as a preferred source of E. coli-produced racemase activity.

B. Demonstration of Isopenicillin N Epimerase (Racemase) Activity in the E. coli K12 JM109/pOW392 Cells Grown at 42° C

Five grams of the induced, pelleted E. coli were mixed with 10 ml of Breaking Buffer (10 mM sodium pyrophosphate-HCl, pH = 8.0 in 5 M urea, 20% glycerol, and 0.1 mM dithiothreitol (DTT)). If the amount of cells after centrifugation was less than 5 gm, the ratio of cells to buffer was maintained.

The cell suspension was then cooled to 2° C in an ice-ethanol bath and was sonicated at full power for 20 seconds. The cooling and sonicating was repeated three times.

Cell debris was removed by centrifugation at 47,000 x g for 20 minutes at 4° C. The supernatant was then filtered through glass wool with the filtrate being the crude enzyme.

The enzyme catalyzes the conversion of isopenicillin N to penicillin N and vice-versa, creating an equilibrium state between the two molecules. Therefore, the activity was measured in two ways, once using isopenicillin N as the initial substrate, and once using penicillin N as the starting material. Total protein was measured by the method of Lowry et al., J. Biol. Chem. 193, 265 (1951). Cell extract was mixed with 1.4 mM isopenicillin N or penicillin N, 0.2 mM dithiothreitol, 0.1 mM pyridoxal 5′-phosphate (as measured by the hydrazine method (Wada, H. and Snell, E., J. Biol. Chem. 236, 2089 (1961)), 50 mM pyrophosphate-HCl, pH = 8.3 in a final volume of 0.5 ml. The reaction mixture was then incubated at 37° C for 20 minutes. The reaction was terminated by boiling for 10 minutes. It was then put through a 0.45 µm filter.

Penicillin N and isopenicillin N are difficult to separate by HPLC. The compounds must be derivatized with o-phthaldialdehyde according to the method of Aswad, D.W., Anal. Biochem. 137, 405 (1984) and Usher, J., Lewis, M., and Hughes, D.W., Anal. Biochem. 149, 105 (1985). Twenty µl of the filtrate were mixed with 5 µl of the derivatizing solution (4 mg o-phthaldialdehyde (Sigma Chemical Co., P.O. Box 14508, St. Louis, MO 63178) dissolved in 300 µl methanol, 250 µl 0.4 M sodium borate, pH = 9.4, 390 µl $H_2O$ and 60 µl 1 M N-acetyl-L-cysteine, adjusted to pH 5-6 with NaOH). The reaction was allowed to proceed for 2 minutes at room temperature before termination with 200 µl 50 mM sodium acetate, pH = 5.0. Fifty µl aliquots were analyzed by HPLC using a flow rate of 1 ml/min and measurement of fluorescence at 360 nm. When isopenicillin N was included in the mixtures the cell extracts expressing the racemase activity converted a portion of isopenicillin to penicillin N. Likewise, penicillin N was converted to isopenicillin N in the presence of these cell extracts. No conversion was seen when the cell extract was not added to the mix. In addition, antibody to the purified racemase enzyme reacted with an ~50,000 dalton band on a Western blot. Taken together, these results indicate that the gene cloned results in a protein which has racemase activity and is the same size as the purified epimerase enzyme.

## Claims

1. Isopenicillin epimerase in substantially purified form which has a molecular weight of 47,000 Daltons as determined by SDS-PAGE; which has an amino acid composition as follows:

| Amino Acid | Number of Residues Per 47,000 Daltons |
|---|---|
| Asx (Asp + Asn) | 29 |
| Thr | 29 |
| Ser | 22 |
| Glx (Glu + Gln) | 41 |
| Pro | 39 |
| Gly | 35 |
| Ala | 58 |
| Val | 21 |
| Cys | 4 |
| Met | 5 |
| Ile | 14 |
| Leu | 56 |
| Tyr | 3 |
| Phe | 14 |
| Lys | 3 |
| His | 9 |
| Arg | 47 |
| Trp | 5 |

which has a 23-residue amino-terminal amino acid sequence as follows: Ala-Val-Ala-Asp-Trp-Glu-Glu-Ala-Arg-Gly-Arg-Met-Leu-Leu-Asp-Pro-Thr-Val-Val-Asn-Leu-Asn-Thr; which exhibits two absorption maxima at 280 nm and 420 nm with absorption coefficients in 10 mM potassium phosphate, pH 7.0, of

$$A_{280}^{1\%} = 8.96 \text{ and } A_{420}^{1\%} = 1.18;$$

which contains one mole of bound pyridoxal-5′-phosphate per mole of enzyme; which on reaction with fluoroescamine forms fluorescent products with maximum excitation and emission wavelength of 390 nm and 475 nm; which exhibits enhanced activity in the presence of sulfhydryl reagents; which exhibits optimal activity at a pH between about 7.8 and about 8.3 in 50 mM pyrophosphate-HCl buffer; which exhibits the following kinetics in the racemization of isopenicillin N and penicillin N,
$V_{max}$ = 3.93 µmole/min/mg (isopenicillin N)
$V_{max}$ = 9.47 µmole/min/mg (penicillin N)
$K_m$ = 0.30 mM (isopenicillin N)
$K_m$ = 0.78 mM (penicillin N);
which contains the 38-residue amino acid sequence bound to pyridoxal-5′-phosphate: Gly-Ile-Thr-Thr-Val-Val-Asp-Gly-Ala-His-Ala-Pro-Gly-Phe-Leu-Asp-Leu-Asp-Leu-Ser-Arg-Ile-Pro-Cys-Asp-Phe-Tyr-Ala-Gly-Phe-Gly-His-Lys-Trp-Leu-Leu-Ala-Pro; and which contains an 11-residue internal amino acid sequence as follows: Leu-Pro-Pro-Gly-Thr-Asp-Ala-Ala-Glu-Leu-Arg.

2. The peptide of the formula Ala-Val-Ala-Asp-Trp-Glu-Glu-Ala-Arg-Gly-Arg-Met-Leu-Leu-Asp-Pro-Thr-Val-Val-Asn-Leu-Asn-Thr.

3. The peptide of the formula Gly-Ile-Thr-Thr-Val-Val-Asp-Gly-Ala-His-Ala-Pro-Gly-Phe-Leu-Asp-Leu-Asp-Leu-Ser-Arg-Ile-Pro-Cys-Asp-Phe-Tyr-Ala-Gly-Phe-Gly-His-Lys-Trp-Leu-Leu-Ala-Pro.

4. The peptide of the formula Leu-Pro-Pro-Gly-Thr-Asp-Ala-Ala-Glu-Leu-Arg.

5. The process for preparing the epimerase of claim 1 in substantially pure form which comprises the steps:

1) adding ammonium sulfate to an aqueous cell-free extract at about pH 8 containing epimerase to achieve a concentration of ammonium sulfate between about 35% and about 70% of saturation and separating the precipitate;

2) dialyzing in the presence of a reducing agent a solution of the precipitate buffered at pH 8;

3) chromatographing the dialysate over a weak anionic exchange resin and eluting epimerase from said resin with a linear gradient of 0.15 M to 0.3 M NaCl in pH 8.0 buffer;

4) pooling the epimerase-containing eluate of step 3 and concentrating said pooled eluate by

ultrafiltration and dialyzing the concentrate at pH 7 in the presence of a reducing agent;

5) chromatographing the dialysate of step 4 over a diethylaminoethyl substituted cross-linked agarose gel and eluting epimerase from said gel with a linear gradient of 0.1 to 0.3 M NaCl in pH 7.0 buffer;

6) concentrating by ultrafiltration the pooled epimerase-containing eluate of step 5 and filtering said concentrate through gel buffered at pH 6.0 in the presence of a reducing agent and pyridoxal-5′-phosphate;

7) concentrating by ultrafiltration the pooled epimerase-containing fractions from step 6, chromatographing the concentrate over calcium phosphate-cellulose at pH 6, and eluting epimerase therefrom with a linear gradient of 10 mM to 100 mM potassium phos phate, pH 6.0, in the presence of a reducing agent and pyridoxal-5′-phosphate;

8) concentrating by ultrafiltration the pooled epimerase-containing eluate from step 7 and dialyzing the concentrate against 10 mM pyrophosphate-HCl at pH 8.0 in the presence of a reducing agent and pyridoxal-5′-phosphate; and

9) chromatographing the dialysate of step 8 buffered at pH 8.0 over a strong anionic resin in the presence of a reducing agent and pyridoxal-5′-phosphate, and eluting said substantially pure epimerase at pH 8.0 with a linear gradient of 0 to 0.4 M NaCl in the presence of a reducing agent and pyridoxal-5′-phosphate; where in steps 1-9 are carried out at a temperature between about 0 °C and about 5 °C.

6. The process of claim 4 wherein the reducing agent is selected from among dithiothreitol, β-mercaptoethanol, and N-acetyl-L-cysteine.

7. The process of claim 4 wherein the pyridoxal-5′-phosphate is present at a concentration of about 10 µM.

8. The process of claim 4 where in step 3 the weak anionic resin is cellulose substituted by diethylaminoethyl groups.

9. The process of claim 4 where in step 9 the strong anionic resin is Mono Q resin.

10. The process of claim 4 where in step 6 the gel is a polysaccharide gel.

Claims for the following Contracting State: ES

1. The process for preparing isopenicillin epimerase in substantially purified form which has a molecular weight of 47,000 Daltons as determined by SDS-PAGE; which has an amino acid composition as follows:

| Amino Acid | Number of Residues Per 47,000 Daltons |
|---|---|
| Asx (Asp + Asn) | 29 |
| Thr | 29 |
| Ser | 22 |
| Glx (Glu + Gln) | 41 |
| Pro | 39 |
| Gly | 35 |
| Ala | 58 |
| Val | 21 |
| Cys | 4 |
| Met | 5 |
| Ile | 14 |
| Leu | 56 |
| Tyr | 3 |
| Phe | 14 |
| Lys | 3 |
| His | 9 |
| Arg | 47 |
| Trp | 5 |

which has a 23-residue amino-terminal amino acid sequence as follows: Ala-Val-Ala-Asp-Trp-Glu-Glu-Ala-Arg-Gly-Arg-Met-Leu-Leu-Asp-Pro-Thr-Val-Val-Asn-Leu-Asn-Thr; which exhibits two absorption maxima at 280 nm and 420 nm with absorption coefficients in 10 mM potassium phosphate, pH 7.0, of

$$A_{280}^{1\%} = 8.96 \text{ and } A_{420}^{1\%} = 1.18;$$

which contains one mole of bound pyridoxal-5′-phosphate per mole of enzyme; which on reaction with fluoroescamine forms fluorescent products with maximum excitation and emission wavelength of 390 nm and 475 nm; which exhibits enhanced activity in the presence of sulfhydryl reagents; which exhibits optimal activity at a pH between about 7.8 and about 8.3 in 50 mM pyrophosphate-HCl buffer; which exhibits the following kinetics in the racemization of isopenicillin N and penicillin N,

$V_{max}$ = 3.93 µmole/min/mg (isopenicillin N)
$V_{max}$ = 9.47 µmole/min/mg (penicillin N)
$K_m$ = 0.30 mM (isopenicillin N)
$K_m$ = 0.78 mM (penicillin N);

which contains the 38-residue amino acid sequence bound to pyridoxal-5′-phosphate: Gly-Ile-Thr-Thr-Val-Val-Asp-Gly-Ala-His-Ala-Pro-Gly-Phe-Leu-Asp-Leu-Asp-Leu-Ser-Arg-Ile-Pro-Cys-Asp-Phe-Tyr-Ala-Gly-Phe-Gly-His-Lys-Trp-Leu-Leu-Ala-Pro; and which contains an 11-residue internal amino acid sequence as follows: Leu-Pro-Pro-Gly-Thr-Asp-Ala-Ala-Glu-Leu-Arg which comprises the steps:

1) adding ammonium sulfate to an aqueous cell-free extract at about pH 8 containing epimerase to achieve a concentration of ammonium sulfate between about 35% and about 70% of saturation and separating the precipitate;

2) dialyzing in the presence of a reducing agent a solution of the precipitate buffered at pH 8;

3) chromatographing the dialysate over a weak anionic exchange resin and eluting epimerase from said resin with a linear gradient of 0.15 M to 0.3 M NaCl in pH 8.0 buffer;

4) pooling the epimerase-containing eluate of step 3 and concentrating said pooled eluate by ultrafiltration and dialyzing the concentrate at pH 7 in the presence of a reducing agent;

5) chromatographing the dialysate of step 4 over a diethylaminoethyl substituted cross-linked agarose gel and eluting epimerase from said gel with a linear gradient of 0.1 to 0.3 M NaCl in pH 7.0 buffer;

6) concentrating by ultrafiltration the pooled epimerase-containing eluate of step 5 and filtering said concentrate through gel buffered at pH 6.0 in the presence of a reducing agent and pyridoxal-5′-phosphate;

7) concentrating by ultrafiltration the pooled epimerase-containing fractions from step 6, chromatographing the concentrate over calcium phosphate-cellulose at pH 6, and eluting epimerase therefrom with a linear gradient of 10 mM to 100 mM potassium phosphate, pH 6.0, in the presence of a reducing agent and pyridoxal-5′-phosphate;

8) concentrating by ultrafiltration the pooled epimerase-containing eluate from step 7 and dialyzing the concentrate against 10 mM pyrophosphate-HCl at pH 8.0 in the presence of a reducing agent and pyridoxal-5′-phosphate; and

9) chromatographing the dialysate of step 8 buffered at pH 8.0 over a strong anionic resin in the presence of a reducing agent and pyridoxal-5′-phosphate, and eluting said substantially pure epimerase at pH 8.0 with a linear gradient of 0 to 0.4 M NaCl in the presence of a reducing agent and pyridoxal-5′-phosphate; where in steps 1-9 are carried out at a temperature between about 0° C and about 5° C.

2. The process of claim 4 wherein the reducing agent is selected from among dithiothreitol, β-mercaptoethanol, and N-acetyl-L-cysteine.

3. The process of claim 4 wherein the pyridoxal-5′-phosphate is present at a concentration of about 10 µM.

4. The process of claim 4 where in step 3 the weak anionic resin is cellulose substituted by diethylaminoethyl groups.

5. The process of claim 4 where in step 6 the gel is a polysaccharide gel.

# FIG.1

## Absorption Spectra of Isopenicillin N Epimerase

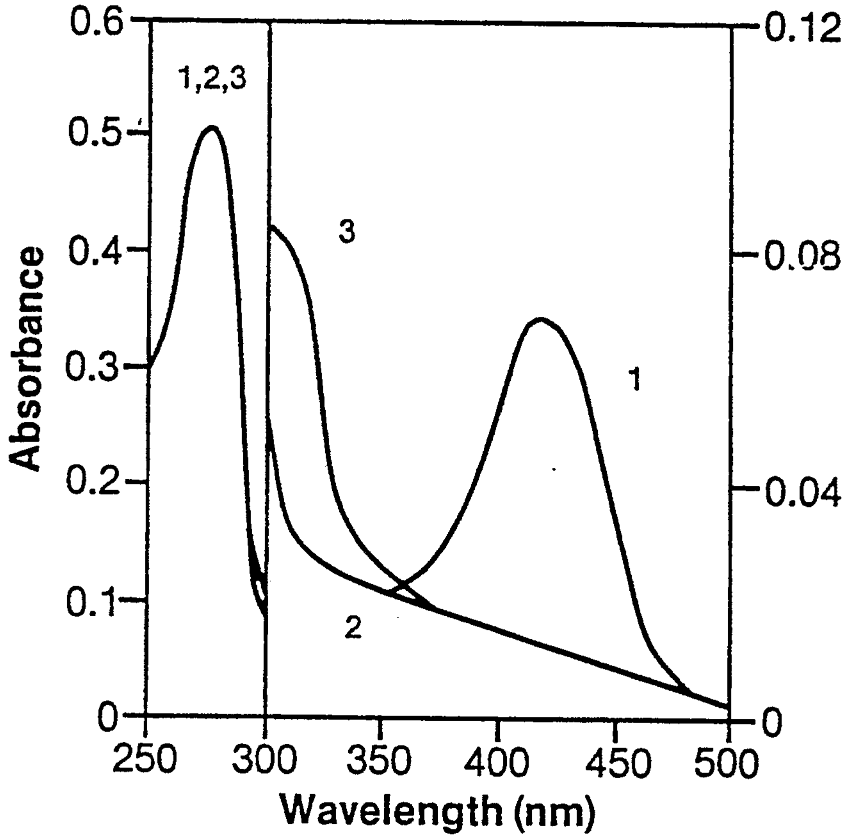

FIG.2

Inhibition and Titration of Isopenicillin N Epimerase by Fluorescamine

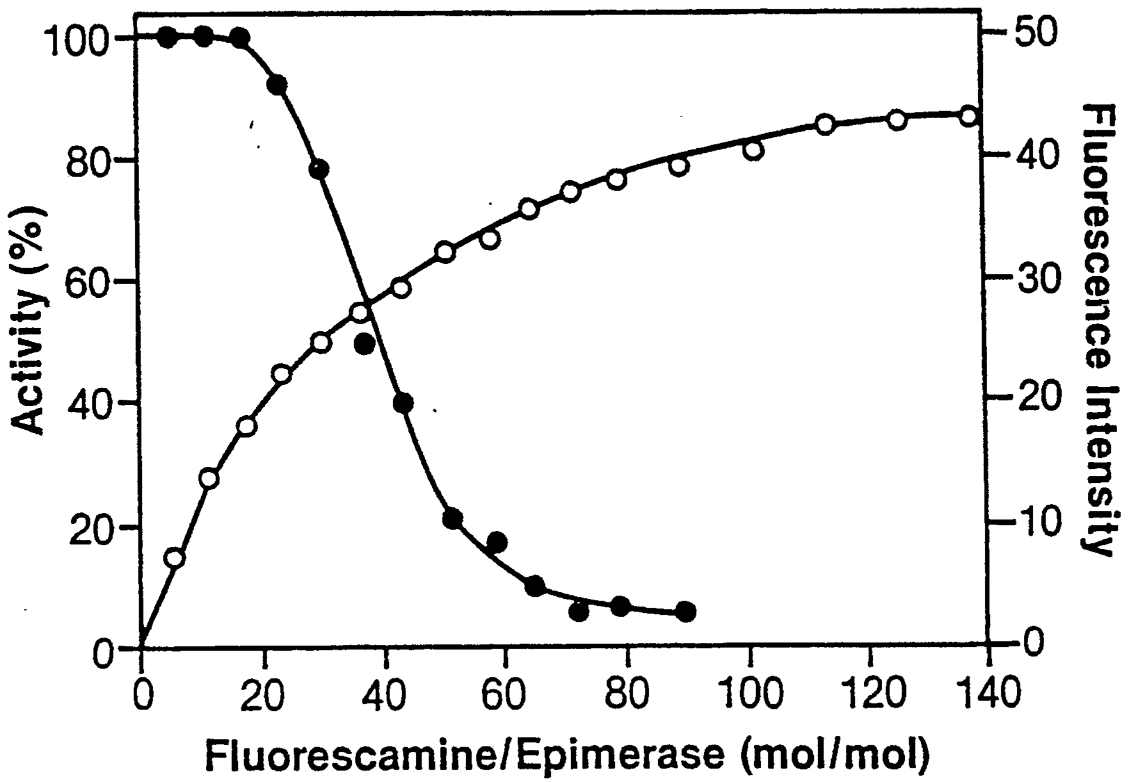

FIG.3

Epimerase SDS-PAGE Electrophoresis Gel and Molecular Weight